(19) Europäisches Patentamt / European Patent Office / Office européen des brevets

(11) **EP 3 522 238 A1**

(12) **EUROPEAN PATENT APPLICATION**
published in accordance with Art. 153(4) EPC

(43) Date of publication:
**07.08.2019 Bulletin 2019/32**

(51) Int Cl.:
**H01L 33/00** (2010.01)   **A23L 3/28** (2006.01)

(21) Application number: **17855889.6**

(86) International application number:
**PCT/JP2017/033911**

(22) Date of filing: **20.09.2017**

(87) International publication number:
**WO 2018/061934 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.09.2016   JP 2016188380**

(71) Applicant: **Nikkiso Co., Ltd.**
**Tokyo 150-6022 (JP)**

(72) Inventor: **MOCHIZUKI Hiroaki**
**Hakusan-shi**
**Ishikawa 924-0004 (JP)**

(74) Representative: **Algemeen Octrooi- en Merkenbureau B.V.**
**P.O. Box 645**
**5600 AP Eindhoven (NL)**

(54) **ULTRAVIOLET IRRADIATION APPARATUS**

(57)    An ultraviolet irradiation device 10 includes: a light source unit 20 that includes at least one ultraviolet LED; a driver 30 that supplies a drive current to the ultraviolet LED; and a controller 60 that controls an operation of the driver 30. The controller 60 calculates a drive current value of the ultraviolet LED based on information indicating spectral intensity characteristics of the ultraviolet LED and information indicating spectral action characteristics of a target of irradiation irradiated by light from the light source unit, and the driver 30 supplies a drive current of a value calculated by the controller 60 to the ultraviolet LED.

FIG. 1

EP 3 522 238 A1

# Description

[TECHNICAL FIELD]

The present invention relates to ultraviolet irradiation devices.

[BACKGROUND ART]

Ultraviolet light is widely used in the field of ultraviolet resin hardening, and for disinfection or sterilization in medical and food processing fronts. For example, an ultraviolet light source for resin hardening is exemplified by one in which an ultraviolet light emitting diode (LED) is used. One such device is configured such that a plurality of ultraviolet LEDs having different wavelength characteristics are combined to address a plurality of types of resin having different hardening wavelengths (see, for example, patent document 1).

[patent document 1] JP2010-56192

[PROBLEM TO BE SOLVED BY THE INVENTION]

Ultraviolet LEDs actually used have individual differences in wavelength characteristics and output intensity, and the target of ultraviolet irradiation have sensitivity characteristics that depend on the wavelength. For this reason, the desired effect may not be obtained or the electric power may be wastefully consumed due to excessive irradiation, unless the wavelength characteristics of the light source and the target of irradiation are properly considered.

In this background, an illustrative purpose of the present invention is to provide an ultraviolet irradiation device capable of irradiating a target of irradiation with ultraviolet light efficiently.

[MEANS TO SOLVE THE PROBLEM]

An ultraviolet irradiation device according to an embodiment of the present invention includes: a light source unit that includes at least one ultraviolet LED; a driver that supplies a drive current to the ultraviolet LED; and a controller that controls an operation of the driver. The controller calculates a drive current value of the ultraviolet LED based on information indicating spectral intensity characteristics of the ultraviolet LED and information indicating spectral action characteristics of a target of irradiation irradiated by light from the light source unit, and the driver supplies a drive current of a value calculated by the controller to the ultraviolet LED.

According to the embodiment, it is possible to estimate an action given by light emission to the target of irradiation, based on the spectral intensity characteristics of the ultraviolet LED and the spectral action characteristics of the target of irradiation and to drive the ultraviolet LED so that the action is optimized. This prevents an insufficient or excessive irradiation level and makes it possible to obtain a desired effect by irradiating the target of irradiation with ultraviolet light efficiently.

The controller may calculate the drive current value of the ultraviolet LED so that an estimated action obtained by integrating a product of the spectral intensity characteristics of the ultraviolet LED and the spectral action characteristics of the target of irradiation over a wavelength meets a predetermined condition.

The controller may calculate the drive current value of the ultraviolet LED based on information indicating correlation between the light emission intensity of the ultraviolet LED and the drive current value of the ultraviolet LED.

The device may further include a measurement unit that measures a light emission intensity of the ultraviolet LED. The measurement unit may calculate the drive current value of the ultraviolet LED based on correlation between a result of measurement by the measurement unit and the drive current value of the ultraviolet LED.

The device may further include an input unit that receives designation of a target value of an action that should be given to the target of irradiation. The controller may calculate the drive current value of the ultraviolet LED to achieve the target value.

The controller may calculate a value indicating a duration of driving the ultraviolet LED to achieve the target value, and the driver may supply the drive current to the ultraviolet LED over the duration of driving of the value calculated by the controller.

The input unit may receive designation of a duration of light irradiation on the target of irradiation, and the controller may calculate the drive current value of the ultraviolet LED so as to meet both the target value of the action and the duration of light irradiation.

The light source unit may include a plurality of ultraviolet LEDs having different spectral intensity characteristics. The controller may calculate a plurality of drive current values corresponding to the plurality of ultraviolet LEDs, respectively, based on information indicating spectral intensity characteristics of the plurality of ultraviolet LEDs, and the driver supplies each of drive currents of a plurality of values calculated by the controller to a corresponding ultraviolet LED.

[ADVANTAGE OF THE INVENTION]

According to the present invention, it is possible to irradiate a target of irradiation with ultraviolet light efficiently.

[BRIEF DESCRIPTION OF THE DRAWINGS]

Fig. 1 schematically shows functions and a configuration of an ultraviolet irradiation device according to an embodiment;

Fig. 2 is a graph schematically showing spectral intensity characteristics of the plurality of LEDs;

Fig. 3 is a graph schematically showing the spectral action characteristics of the target of irradiation;

Fig. 4 is a graph schematically showing examples of calculating the coefficients indicating the light emission intensity of the LEDs; and

Fig. 5 is a graph schematically showing the correlation between the light emission intensity of the LED and the drive current value I.

[MODE FOR CARRYING OUT THE INVENTION]

A detailed description will be given of embodiments of the present invention with reference to the drawings. Like numerals are used in the description to denote like elements and a duplicate description is omitted as appropriate.

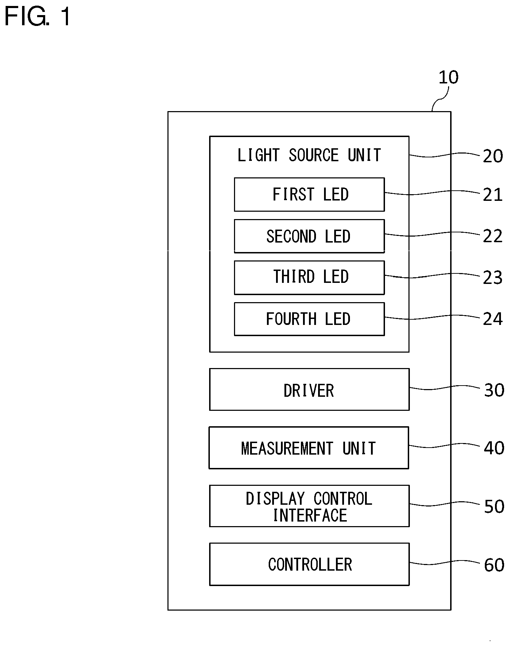

Fig. 1 schematically shows functions and a configuration of an ultraviolet irradiation device 10 according to an embodiment. The ultraviolet irradiation device 10 includes a light source unit 20, a driver 30, a measurement unit 40, a display control interface 50, and a controller 60. The ultraviolet irradiation device 10 is used for sterilization and resin hardening by ultraviolet irradiation. For example, the ultraviolet irradiation device 10 may be used by being built in a fluid sterilization device for irradiating a fluid such as water with ultraviolet light to sterilize the fluid continuously.

The light source unit 20 includes a plurality of LEDs 21, 22, 23, and 24. At least one of the plurality of LEDs 21, 22, 23, and 24 is configured to output deep ultraviolet light having a central wavelength or a peak wavelength included in a range of about 250 nm ~ 350 nm. Such an ultraviolet LED is exemplified by an aluminum gallium nitride (AlGaN) based LED. At least one of the plurality of LEDs 21-24 may be configured to output ultraviolet light or blue light having a central wavelength or a peak wavelength included in a range of about 350 nm ~ 450 nm. Such an ultraviolet or blue LED is exemplified by a gallium nitride (GaN) based LED. The light source unit 20 may include an LED for emitting visible light or infrared light having a wavelength longer than 450 nm. In this embodiment, the case of including four LEDs is illustrated, but the embodiment is non-limiting as to the number of LEDs included in the light source unit 20. The light source unit 20 may include a plurality of LEDs having substantially identical wavelength characteristics.

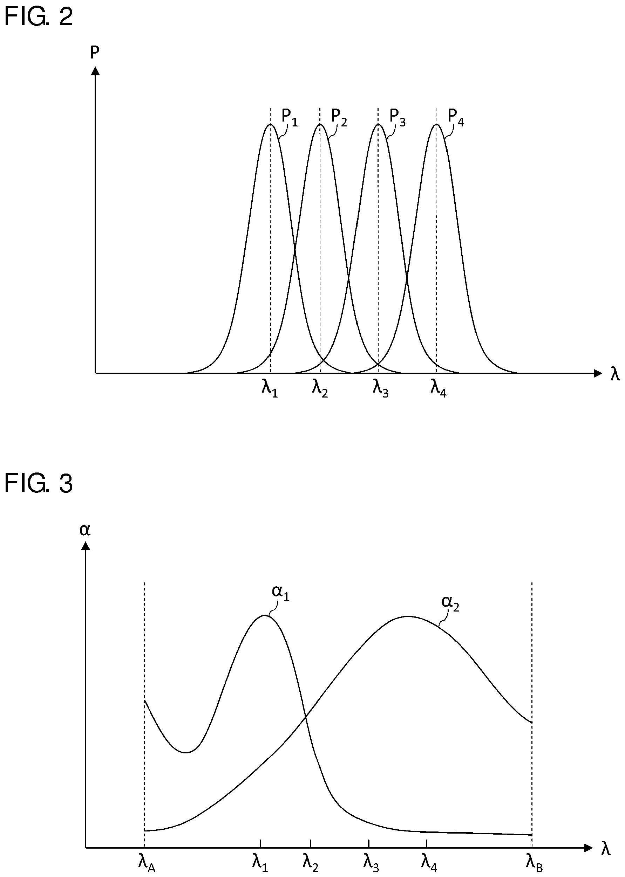

Fig. 2 is a graph schematically showing spectral intensity characteristics of the plurality of LEDs 21 ~ 24. The plurality of LEDs 21 ~ 24 has mutually different wavelength characteristics. The first LED 21 has the first intensity distribution $P_1(\lambda)$ in which the central wavelength or the peak wavelength is the first wavelength $\lambda_1$. The first LED 22 has the second intensity distribution $P_2(\lambda)$ in which the central wavelength or the peak wavelength is the second wavelength $\lambda_2$. The third LED 23 has the third intensity distribution $P_3(\lambda)$ in which the central wavelength or the peak wavelength is the third wavelength $\lambda_3$. The fourth LED 24 has the fourth intensity distribution $P_4(\lambda)$ in which the central wavelength or the peak wavelength is the fourth wavelength $\lambda_4$.

In one embodiment, the wavelength characteristics of the plurality of ultraviolet LEDs 21 ~ 24 are configured to have central wavelengths or peak wavelengths such that $\lambda_1 < \lambda_2 < \lambda_3 < \lambda_4$. Further, the wavelength characteristics are selected such that the intensity distributions of LEDs having adjacent central wavelengths or peak wavelengths overlap each other. In this case, the ultraviolet irradiation device 10 is configured to output ultraviolet light having a continuous spectrum in a wavelength range at least from the first wavelength $\lambda_1$ to the fourth wavelength $\lambda_4$. In one variation, the wavelength characteristics may be selected such that the intensity distributions of LEDs having adjacent central wavelengths or peak wavelengths do not overlap. In this case, the device is configured not to output ultraviolet light in a certain limited wavelength range.

It is preferred that the plurality of wavelength characteristics of LEDs 21 ~ 24 is selected in accordance with the usage of the ultraviolet irradiation device 10. In the case the ultraviolet irradiation device 10 is used for the purpose of sterilization, for example, it is preferred to include an LED capable of outputting ultraviolet light in a wavelength range of about 260 ~ 270 nm, which is known to have high sterilization capability. In the case the ultraviolet irradiation device 10 is used for resin hardening, it is preferred to include an LED capable of outputting ultraviolet light in a wavelength range of about 300 ~ 350 nm or a wavelength range of about 350 nm ~ 400 nm, depending on the resin hardening wavelength.

Referring back to Fig. 1, the driver 30 is configured to supply a drive current to the plurality of LEDs 21 ~ 24 included in the light source unit 20. For example, the driver 30 includes a constant-current circuit for supplying a constant current to the plurality of LEDs 21 ~ 24. The driver 30 is configured to supply drive currents of different values to the plurality of LEDs 21-24, respectively, in accordance with an instruction from the controller 60. The driver 30 is capable of controlling the light emission intensity of the plurality of LEDs 21 ~ 24 independently.

The measurement unit 40 measures the output intensity of the light source unit 20 and transmits a result of measurement to the controller 60. The measurement unit 40 includes, for example, a power meter capable of measuring the light intensity. By providing the measurement unit 40, it is possible to monitor the output of the light source unit 20 and perform feedback control to maintain the output intensity of the light source unit 20 constant.

The measurement unit 40 may be configured to measure the spectral intensity characteristics of the light source unit 20 and may, for example, include a spectrometer. The measurement unit 40 may generate information related to the spectral intensity characteristics as shown in Fig. 2 by measuring the spectral characteristics of the output light of the LEDs 21 ~ 24. The measurement unit 40 may be configured to measure the wavelength sensitivity of the target of irradiation. For example, the measurement unit 40 may be configured to measure the wavelength dependency of the absorption of light by the target of irradiation.

The display control interface 50 is an input unit for receiving a user operation from a user. For example, the display control interface 50 is comprised of a touch-sensitive panel device. The display control interface 50 displays a screen in which to enter or select an operating condition of the ultraviolet irradiation device 10 and allows the user to enter an operating condition of the ultraviolet irradiation device 10. The display control interface 50 may be comprised of a display unit and an input unit that are separate from each other.

For example, the display control interface 50 makes it possible to enter and configure parameters related to the target of irradiation or parameters related to the irradiation condition. Parameters related to the target of irradiation are exemplified by the type, quantity, density, etc. of the target of irradiation. The interface may receive an input of information related to the wavelength sensitivity (spectral action characteristics described later, etc.; see Fig. 3) of the target of irradiation, as a parameter related to the target of irradiation. The interface may receive an input of information related to the processing duration, the total amount of irradiation energy (total dose), the target value of action given to the target of irradiation, as parameters related to the irradiation condition.

The display control interface 50 may allow the user to select one of a plurality of irradiation modes that are made available. For example, a low power consumption mode, a short duration mode, an automatic mode, etc. may be made available. In the low power consumption mode, the drive current value is determined so that, for example, the power consumption required for the target action is minimized. In the short duration mode, the drive current value is determined so that, for example, the irradiation duration to obtain the target action is minimized. In the automatic mode, the drive current value is determined so that, for example, both the power consumption and the irradiation duration are optimized.

The display control interface 50 may allow the user to select one of a plurality of targets of irradiation available. In the case the device is used for sterilization, for example, a combination of a fluid subject to sterilization and a bacterial strain sought to be sterilized may be designated. In the case the device is used for resin hardening, the resin material subject to irradiation may be designated.

The controller 60 calculates drive current values of the plurality of LEDs 21 ~ 24 based on the spectral intensity characteristics of the plurality of LEDs 21 ~ 24 and the spectral action characteristics of the target of irradiation. The controller 60 maintains the information on the spectral intensity characteristics and the information on the spectral action characteristics. The controller 60 identifies the spectral action characteristics that should be referred to, based on an input in the display control interface 50. The controller 60 estimates the action given to the target of irradiation exposed to light, based on the spectral intensity characteristics of the plurality of LEDs 21 ~ 24 and the spectral action characteristics of the target of irradiation. The controller 60 determines the light emission intensity of the LEDs 21 ~ 24 so that the estimated action meets a predetermined condition and determines drive current values necessary to obtain the determined light emission intensity.

The action given to the target of irradiation exposed to light is a numerical degree of the desired effect expected to be obtained by ultraviolet irradiation. An action E is given by the following expression (1), using the spectral intensity distribution $P(\lambda)$ of the light source unit 20, the spectral action characteristics $\alpha(\lambda)$ of the target of irradiation, and the duration t of ultraviolet irradiation. In other words, the action E is obtained by integrating, over the wavelength, the spectral intensity distribution $P(\lambda)$ of the light source unit 20 as a whole and the spectral action characteristics $\alpha(\lambda)$ of the target of irradiation. The wavelengths $\lambda_A$, $\lambda_B$ defining the range of integration correspond to the lower limit value and the upper limit value of the wavelength range in which the light source is capable of outputting light.

$$ E = t \int_{\lambda_A}^{\lambda_B} \alpha(\lambda) P(\lambda) \mathrm{d}\lambda \qquad \cdots (1) $$

The spectral action characteristics $\alpha(\lambda)$ of the target of irradiation is the wavelength dependency or the wavelength sensitivity with respect to the degree of the effect obtained by ultraviolet irradiation. For example, the spectral action characteristics in the case of sterilization represents the correlation between the wavelength $\lambda$ of ultraviolet light and the rate of sterilization, and the spectral action characteristics in the case of resin hardening represents correction between the wavelength $\lambda$ of ultraviolet light and the level of resin hardening by exposure to ultraviolet light.

Fig. 3 is a graph schematically showing the spectral action characteristics of the target of irradiation. The graph illustrates the spectral action characteristics $\alpha_1(\lambda)$ in sterilization and the spectral characteristics $\alpha_2(\lambda)$ in resin hardening. The spectral action characteristics $\alpha_1(\lambda)$ in sterilization has a curved form in which, for example, the level of sterilization is at maximum near $\lambda=260$ nm. The spectral action characteristics $\alpha_2(\lambda)$ in sterilization has a curved form in which, for example, the level of resin hardening is at maximum near $\lambda=330$ nm. The graph shown is for an illustrative purpose only, and it will be understood that the graph form varies depending on the type of the target of irradiation or the action sought to be obtained by ultraviolet irradiation.

The controller 60 determines the light emission intensity of the LEDs 21 ~ 24 so that the action E obtained is maximized, based on the spectral intensity characteristics $P_1(\lambda)$ - $P_4(\lambda)$ of the LEDs 21 ~ 24 and the spectral action characteristics $\alpha(\lambda)$ of the target of irradiation. More specifically, the coefficients $k_i$ (i:1 ~ 4) indicating the relative values of light emission intensity of the respective LEDs are determined so that the estimated action ER per unit time calculated by using the following expression (2) is maximized. For example, the value of the coefficient $k_i$ indicating the light emission of each LED is determined by a solving known optimization problem under the condition that the sum of the coefficients $k_i$ is constant (e.g., $k_1+k_2+k_3+k_4=1$).

$$E_R = \sum_{i=1}^{n} \int_{\lambda_A}^{\lambda_B} \alpha(\lambda) k_i P_i(\lambda) \mathrm{d}\lambda \quad \cdots (2)$$

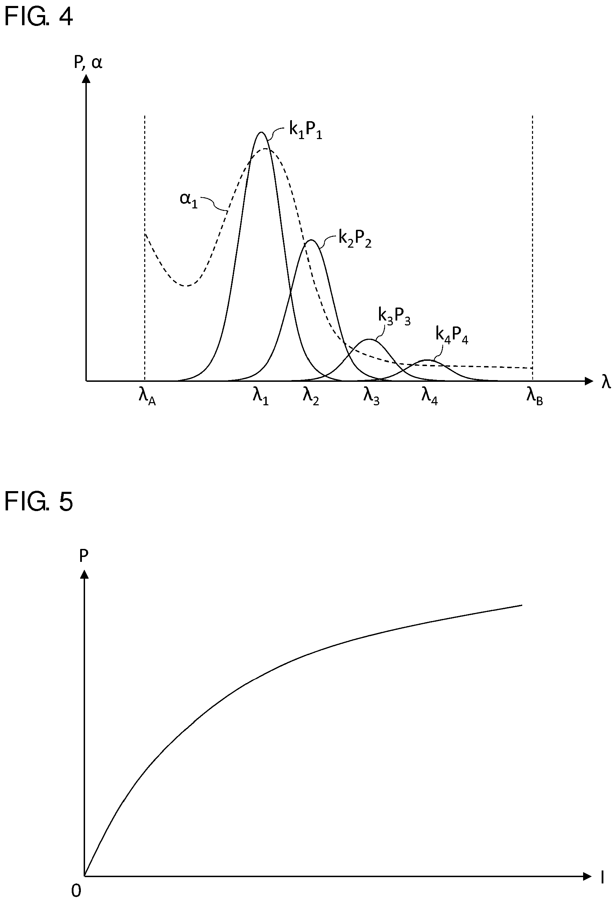

Fig. 4 is a graph schematically showing examples of calculating the coefficients $k_1$ ~ $k_4$ indicating the light emission intensity of the respective LEDs. In the illustrated example, the coefficient $k_i$ of the light intensity of each LED is calculated based on predetermined spectral action characteristics $\alpha_1(\lambda)$. In the illustrated example, the coefficients $k_i$ are determined such that the coefficient $k_1$ of the first LED 21 corresponding to the first wavelength $\lambda_1$ having a high value in the spectral action characteristics $\alpha_1(\lambda)$ is large, and the coefficient $k_4$ of the fourth LED 24 corresponding to the fourth wavelength $\lambda_4$ having a low value in the spectral action characteristics $\alpha_1(\lambda)$ is small. In other words, the coefficients $k_i$ are determined such that the higher the contribution of the LED to the spectral action characteristics $\alpha$, the higher the light mission intensity. Thus, by ensuring that the light emission intensity is large at the wavelength $\lambda$ characterized by large spectral action characteristics $\alpha$ and that the light emission intensity is small at the wavelength $\lambda$ characterized by small spectral action characteristics $\alpha$, the action E given to the target of irradiation is increased and the power consumption in the light source as a whole is decreased.

The controller 60 calculates the drive current values I1, I2, I3, and I4 of the LEDs 21 ~ 24 to obtain the light intensity in accordance with the coefficients $k_i$ determined. The controller 60 maintains information indicating the correlation between the light intensity P and the drive current value I of the LED and determines the drive current value of each LED based on the correlation information. Fig. 5 is a graph schematically showing the correlation between the light emission intensity P and the drive current value I of the LED. By referring to the correlation as illustrated, it is possible to determine the drive current value I corresponding to the given light emission intensity P. In one variation, the drive current Ii (i: 1 - 4) may be calculated in a simplified manner by multiplying the coefficient $k_i$ indicating the light emission intensity $k_i$ by a predetermined constant. Alternatively, the drive current value Ii may be calculated based on a result of measurement by the measurement unit 40.

The controller 60 may subject the drive current values I1 ~ I4 of the LEDs 21 ~ 24 to feedback control so as to maintain the light emission intensity in accordance with the coefficient $k_i$ determined. For example, the drive current determined from the correlation between the light intensity emission and the drive current value shown in Fig. 5 may be defined as an initial value, and the light emission intensity of the LEDs 21 ~ 24 driven by the initial value is measured by the measurement unit 40. Subsequently, the drive current value is determined in such a manner as to maintain the light emission intensity thus measured.

The controller 60 may calculate the values of the coefficients $k_i$ indicating the light emission intensity of the respective LEDs so that the estimated action ER per unit time reaches a predetermined target value ET. The target value ET related to the action per unit time may be configured through an input via the display control interface 50, or the controller 60 may calculate the target value ET based on another parameter input via the display control interface 50. For example, the target value Et may be calculated based on parameters entered by the user such as the type of target of irradiation, the quantity or density of the target of irradiation, and the duration of irradiation. In the case that the target of irradiation is a fluid, the target value ET may be calculated based on information related to the flow rate.

When a plurality of solutions for combinations of coefficients $k_i$ are obtained, the controller 60 may calculate the combination that minimizes the sum of the coefficients $k_i$ as the solution. Alternatively, the controller 60 may calculate the solution of the coefficients $k_i$ so that the sum of the drive current values I1 ~ I4 of the LEDs 21 ~ 24 is minimized. In this process, the controller 60 may use the information indicating the correlation between the light emission intensity and the drive current values of the LEDs 21 ~ 24 as shown in Fig. 5. The controller 60 may further maintain information indicating the maximum current value capable of driving the LEDs 21 ~ 24 and calculate the solution for the coefficients $k_i$ by using the maximum current value as a constraint condition. For example, the controller 60 may calculate the solution for the coefficients $k_i$ so that the maximum current value is induced at least in one of the plurality of LEDs 21 ~ 24.

The controller 60 may determine the light emission intensity of the LEDs based on a target value related to the duration of processing. for example, the controller 60 may calculate an action ER per nit time necessary to obtain a target value ET of the action within a predetermined duration of irradiation and may determine the light emission intensity of the LEDs so that the calculated action ER is obtained. In this case, the controller 60 may calculate a coefficient $k_i$ different from the coefficient $k_i$

of the light intensity calculated to minimize the power consumption. For example, the controller 60 may control the LEDs to be driven such that the target action ET is obtained in the shortest period of time at a certain cost of loss in irradiation efficiency.

The controller 60 may determine the duration t of ultraviolet irradiation based on a target value of the total amount of irradiation energy (total dose). For example, the controller 60 may calculate a target value EU of the total action necessary for processing by referring to the information such as the type, quantity, density of the target of irradiation and determine the duration of irradiation according to the expression t=EU/ER, using the estimated action ER per unit time. The controller 60 may estimate the dose irradiating the target of irradiation based on the light emission intensity measured by the measurement unit 40 and determine the time required for the total dose to reach the target value as the duration of irradiation.

A description will be given of the operation of the ultraviolet irradiation device 10 configured as described above. The ultraviolet irradiation device 10 first acquires the information related to the spectral intensity characteristics of the light source unit 20 and the information related to the spectral action characteristics of the target of irradiation. These items of information may be acquired from the measurement unit 40, stored in advance in the controller 60, entered via the display control interface 50, or acquired from an external device such as a database connected via a network.

The ultraviolet irradiation device 10 then receives configuration of parameters related to the target of irradiation and to the irradiation condition. The controller 60 determines the coefficients $k_i$ indicating the relative values of light emission intensity of the LEDs 21 ~ 24 based on the parameters configured and calculates the drive current values Ii of the LEDs 21 ~ 24 to realize the coefficients $k_i$. The controller 60 may also calculate the duration of irradiation. The driver 30 supplies the calculated drive current values Ii to the associated LEDs 21 ~ 24 to drive the LEDs 21 ~ 24 in a light emission intensity ratio in accordance with the coefficients $k_i$. The controller 60 may stop driving the LEDs 21 ~ 24 after an elapse of a predetermined duration of irradiation, and the display control interface 50 displays a message indicating that the irradiation process is completed.

According to the embodiment, the LED can be driven so that the action given to the target of irradiation is optimized by determining the drive current values of the LEDs 21 ~ 24 based on the spectral intensity characteristics of the LEDs 21 ~ 24 and the spectral action characteristics of the target of irradiation. The ultraviolet LED used in the light source unit 20 has a central wavelength or a peak wavelength as design values and a spread width (e.g., a full width at half maximum) of the wavelength distribution, but the wavelength characteristics vary between the individual LEDs that are actually used. If the light emission intensity or the duration of irradiation is determined without regard to the wavelength characteristics of individual ultraviolet LEDs, therefore, the irradiation level may become insufficient or excessive, which may result in failure to realize efficient ultraviolet irradiation. According to the embodiment, on the other hand, control based on the spectral intensity characteristics of individual LEDs and the spectral action characteristics of the target of irradiation is performed so that it is possible to drive the LEDs in a condition optimized for the irradiation process. This improves the efficiency of ultraviolet irradiation.

Described above is an explanation based on an exemplary embodiment. The embodiment is intended to be illustrative only and it will be understood by those skilled in the art that various design changes are possible and various modifications are possible and that such modifications are also within the scope of the present invention.

In the embodiments described above, the light source unit 20 is described as including a plurality of LEDs. In one variation, the light source unit 20 may include only one LED. It is also possible, in this case, to make the irradiation process efficient by determining the drive current values based on both the wavelength characteristics of the one ultraviolet LED and the wavelength characteristics of the target of irradiation.

[DESCRIPTION OF THE REFERENCE NUMERALS]

10 ... ultraviolet irradiation device, 20 ... light source unit, 21 ... first LED, 22 ... second LED, 23 ... third LED, 24 ... fourth LED, 30 ... driver, 40 ... measurement unit, 50 ... display control interface, 60 ... controller

[INDUSTRIAL APPLICABILITY]

According to the invention, a target of irradiation is irradiated with ultraviolet light efficiently.

**Claims**

1. An ultraviolet irradiation device comprising:

   a light source unit that includes at least one ultraviolet LED;
   a driver that supplies a drive current to the ultraviolet LED; and
   a controller that controls an operation of the driver, wherein
   the controller calculates a drive current value of the ultraviolet LED based on information indicating spectral intensity characteristics of the ultraviolet LED and information indicating spectral action characteristics of a target of irradiation irradiated by light from the light source unit, and
   the driver supplies a drive current of a value calculated by the controller to the ultraviolet LED.

**2.** The ultraviolet irradiation device according to claim 1, wherein
the controller calculates the drive current value of the ultraviolet LED so that an estimated action obtained by integrating a product of the spectral intensity characteristics of the ultraviolet LED and the spectral action characteristics of the target of irradiation over a wavelength meets a predetermined condition.

**3.** The ultraviolet irradiation device according to claim 1 or 2, wherein
the controller calculates the drive current value of the ultraviolet LED based on information indicating correlation between the light emission intensity of the ultraviolet LED and the drive current value of the ultraviolet LED.

**4.** The ultraviolet irradiation device according to claim 1 or 2, further comprising:

a measurement unit that measures a light emission intensity of the ultraviolet LED, wherein
the measurement unit calculates the drive current value of the ultraviolet LED based on correlation between a result of measurement by the measurement unit and the drive current value of the ultraviolet LED.

**5.** The ultraviolet irradiation device according to any one of claims 1 through 4, further comprising:

an input unit that receives designation of a target value of an action that should be given to the target of irradiation, wherein
the controller calculates the drive current value of the ultraviolet LED to achieve the target value.

**6.** The ultraviolet irradiation device according to claim 5, wherein
the controller calculates a value indicating a duration of driving the ultraviolet LED to achieve the target value, and
the driver supplies the drive current to the ultraviolet LED over the duration of driving of the value calculated by the controller.

**7.** The ultraviolet irradiation device according to claim 5 or 6, wherein
the input unit receives designation of a duration of light irradiation on the target of irradiation, and
the controller calculates the drive current value of the ultraviolet LED so as to meet both the target value of the action and the duration of light irradiation.

**8.** The ultraviolet irradiation device according to any one of claims 1 through 7, wherein
the light source unit includes a plurality of ultraviolet LEDs having different spectral intensity characteristics,
the controller calculates a plurality of drive current values corresponding to the plurality of ultraviolet LEDs, respectively, based on information indicating spectral intensity characteristics of the plurality of ultraviolet LEDs, and
the driver supplies each of drive currents of a plurality of values calculated by the controller to a corresponding ultraviolet LED.

FIG. 1

10

| | |
|---|---|
| **LIGHT SOURCE UNIT** | 20 |
| FIRST LED | 21 |
| SECOND LED | 22 |
| THIRD LED | 23 |
| FOURTH LED | 24 |
| DRIVER | 30 |
| MEASUREMENT UNIT | 40 |
| DISPLAY CONTROL INTERFACE | 50 |
| CONTROLLER | 60 |

FIG. 2

FIG. 3

FIG. 4

FIG. 5

**INTERNATIONAL SEARCH REPORT**

| International application No. |
|---|
| PCT/JP2017/033911 |

A. CLASSIFICATION OF SUBJECT MATTER
*H01L33/00*(2010.01)i, *A23L3/28*(2006.01)i

According to International Patent Classification (IPC) or to both national classification and IPC

B. FIELDS SEARCHED

Minimum documentation searched (classification system followed by classification symbols)
H01L33/00-33/64, A23L3/28, A61L2/00-2/28, A61L11/00-12/14, F21K9/00-9/90, F21S2/00-19/00, H01L21/30, H01L21/46, B05C7/00-21/00

Documentation searched other than minimum documentation to the extent that such documents are included in the fields searched
Jitsuyo Shinan Koho          1922-1996   Jitsuyo Shinan Toroku Koho   1996-2017
Kokai Jitsuyo Shinan Koho    1971-2017   Toroku Jitsuyo Shinan Koho   1994-2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2015-193002 A  (Kyocera Corp.),<br>05 November 2015 (05.11.2015),<br>paragraphs [0002], [0012] to [0086]; fig. 1 to 11<br>(Family: none) | 1,3,8<br>2,4-7 |
| Y | JP 2003-25707 A  (Konica Corp.),<br>29 January 2003 (29.01.2003),<br>paragraphs [0042], [0056], [0063] to [0078]<br>(Family: none) | 2,4-7 |
| Y | WO 2015/199148 A1  (Nissan Chemical Industries, Ltd., Kyusyu Nanotec Optics Co., Ltd.),<br>30 December 2015 (30.12.2015),<br>paragraphs [0096] to [0107]<br>& EP 3163367 A1          & US 2017/0199434 A1<br>paragraphs [0184] to [0202] | 4-7 |

| ☒   Further documents are listed in the continuation of Box C. | ☐   See patent family annex. |
|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>     13 October 2017 (13.10.17) | Date of mailing of the international search report<br>     07 November 2017 (07.11.17) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

## INTERNATIONAL SEARCH REPORT

| International application No. |
|---|
| PCT/JP2017/033911 |

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2006-256322 A  (Keyence Corp.),<br>28 September 2006 (28.09.2006),<br>paragraphs [0025] to [0114]; fig. 1 to 24<br>& JP 3811862 B1 | 5-7 |
| Y | JP 2013-84691 A  (Sharp Corp.),<br>09 May 2013 (09.05.2013),<br>paragraphs [0083] to [0101]<br>(Family: none) | 6-7 |
| Y | JP 2008-307754 A  (Tohoku Ricoh Co., Ltd.),<br>25 December 2008 (25.12.2008),<br>paragraphs [0047] to [0055]<br>(Family: none) | 6-7 |
| P,X | JP 2017-43079 A  (Kyocera Corp.),<br>02 March 2017 (02.03.2017),<br>paragraphs [0002], [0012] to [0084]; fig. 1 to 7<br>(Family: none) | 1,3,8 |
| A | JP 2012-51335 A  (NK Works Co., Ltd.),<br>15 March 2012 (15.03.2012),<br>paragraphs [0026] to [0061]; fig. 1 to 7<br>& WO 2012/029198 A1 | 1-8 |
| A | US 2006/0204670 A1  (SIEGEL),<br>14 September 2006 (14.09.2006),<br>paragraphs [0041] to [0094]; fig. 1 to 21<br>& US 2004/0135159 A1    & US 2006/0121208 A1<br>& US 2004/0166249 A1    & US 2007/0139504 A1<br>& WO 2007/090049 A1 | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010056192 A